(19) Europäisches Patentamt European Patent Office Office européen des brevets

(11) **EP 4 506 194 A1**

(12) **EUROPEAN PATENT APPLICATION**

(43) Date of publication:
**12.02.2025 Bulletin 2025/07**

(21) Application number: **23315307.1**

(22) Date of filing: **08.08.2023**

(51) International Patent Classification (IPC):
**B60H 3/06** $^{(2006.01)}$  **G01N 15/06** $^{(2024.01)}$

(52) Cooperative Patent Classification (CPC):
**B60H 1/008; G01N 15/06;** B60H 3/06;
G01N 33/0037; G01N 2015/0046

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **MANN+HUMMEL Ventures Pte. Ltd. Singapore 139234 (SG)**

(72) Inventors:
• **Woitoll, Ina 71636 Ludwigsburg (DE)**

• **Kunze, Stefan 71636 Ludwigsburg (DE)**
• **Zenner, Julien 71636 Ludwigsburg (DE)**
• **Michel, Christiane 71636 Ludwigsburg (DE)**

(74) Representative: **Mann + Hummel Intellectual Property Mann + Hummel International GmbH & Co. KG Schwieberdinger Straße 126 71636 Ludwigsburg (DE)**

(54) **DEVICE AND METHOD FOR ESTIMATING AN ULTRA-FINE PARTICLE CONCENTRATION**

(57) Aspects concern a method for estimating an ultra-fine particle concentration, comprising receiving data indicative of, a measured ultra-fine particle concentration and a measured nitrogen oxide concentration, the measured ultra-fine particle concentration and the measured nitrogen oxide concentration correlated to each location of a plurality of locations and corresponding time of a plurality of times; establishing a correlation between ultra-fine particle concentration and nitrogen oxide concentration from the measured ultra-fine particle concentrations and the measured nitrogen oxide concentrations; and estimating, from a nitrogen oxide concentration measured at a location where the ultra-fine particle concentration is to be estimated, the ultra-fine particle concentration according to the correlation.

500

501

Receive data indicative of, for each of a plurality of locations and each of a plurality of times, a measured ultra fine particle concentration and a measured nitrogen oxide concentration

502

Establish a correlation between ultra fine particle concentration and nitrogen oxide concentration from the measured ultra fine particle concentrations and the measured nitrogen oxide concentrations

503

Estimate, from a nitrogen oxide concentration measured at a location where the ultra fine particle concentration is to be estimated, the ultra fine particle concentration according to the correlation

**FIG 5**

EP 4 506 194 A1

**Description**

## TECHNICAL FIELD

Various aspects of this disclosure relate to devices and methods for estimating an ultra-fine particle concentration.

## BACKGROUND

For a vehicle, it may be desirable to have information about ultra-fine particles (UFP) in its surrounding air, e.g. for controlling a cabin air system.

While air quality stations exist that may provide information about pollution on the street level, those may not cover every location, at least not for all types of pollution. For example, a certain (local) measurement station may only provide information about a current (local) pollution status regarding defined parameters (such as PM10, PM2.5, or gas concentration) which thus may in particular not cover the concentration of ultra-fine particles. Moreover, measuring ultra-fine particle concentration is difficult and requires high technical effort so it is typically not desirable to equip vehicles with sensors that measure ultra-fine particle concentration.

Accordingly, efficient and accurate approaches for estimating ultra-fine particle concentration are desirable.

## SUMMARY

Various embodiments concern a method for estimating an ultra-fine particle concentration, comprising receiving data indicative of, for each of a plurality of locations and each of a plurality of times, a measured ultra-fine particle concentration and a measured nitrogen oxide concentration, the measured ultra-fine particle concentration and the measured nitrogen oxide concentration correlated to each location of a plurality of locations and corresponding time of a plurality of times; establishing a correlation between ultra-fine particle concentration and nitrogen oxide concentration from the measured ultra-fine particle concentrations and the measured nitrogen oxide concentrations; and estimating, from a nitrogen oxide concentration measured at a location where the ultra-fine particle concentration is to be estimated, the ultra-fine particle concentration according to the correlation.

In some embodiments, the nitrogen concentration is a nitrogen monoxide concentration, a nitrogen dioxide concentration, or a concentration of nitrogen monoxide and nitrogen dioxide. The various nitrogen dioxide concentration may be referred hereinafter as (i.e. a NOx concentration).

In some embodiments, the method further comprises receiving, by a server, a request for an estimate of the ultra-fine particle concentration, wherein the request indicates the nitrogen oxide concentration, and providing the estimated ultra-fine particle concentration in response to the request.

The request may be received by a server computer or server computer arrangement, e.g. such as a cloud server for performing the method.

In some embodiments, the method further comprises measuring, by a vehicle sensor, the nitrogen oxide concentration at the location where the ultra-fine particle concentration is to be estimated.

In some embodiments, the method further comprises requesting the nitrogen oxide, concentration at the location where the ultra-fine particle concentration is to be estimated from a database.

In some embodiments, the method further comprises controlling a vehicle system using the estimated ultra-fine particle concentration. In some embodiments, the vehicle system may be a cabin air system.

In some embodiments, controlling the cabin air system comprises increasing a filtration strength in response to that the estimated ultra-fine particle concentration indicates that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased. The increasing of the filter strength may mean increasing an air airflow through a filter element. The increased airflow may be achieved via increasing a blower speed of a suction device, and/or opening one or more airflow control flaps.

In some embodiments, estimating the ultra-fine particle concentration further comprises depending on the location where the ultra-fine particle concentration is to be estimated, a time parameter, and information about a weather at the location where the ultra-fine particle concentration is to be estimated.

According to one embodiment, the method comprises requesting the NOx concentration at the location where the ultra-fine particle concentration is to be estimated from a database. For example, electronic data of a map of UFP concentrations which may be calculated from NOx concentrations, or the NOx concentrations data, may be obtained and/or updated continuously and from the map, the UFP concentration, or an estimation of the UFP concentration based on the stored NOx concentration may be obtained. So, a vehicle does not need to have a NOx sensor and GPS coordinates may be sufficient to send from the vehicle to a server that gathers the NOx concentration elsewhere. The gathered NOx concentration may then be used by the server or the vehicle to derive the UFP concentration.

According to another aspect of the disclosure there is provided a data processing system configured to perform the aforementioned method.

According to another aspect of the disclosure there is provided a cabin air system controller for a cabin air system of a vehicle, the cabin air system controller comprising at least one processor configured to obtain a measurement of a nitrogen oxide concentration at a location of the vehicle; sending a request, from the controller, to a server, for an estimate of an ultra-fine particle concentration based on the nitrogen oxide concentration; receive, by the controller, an estimated ultra-fine particle concentration in response to the request, the estimated ultra-fine particle concentration determined according to any one of the aforementioned method; and control a filtration system of the cabin air system based on the estimated ultra-fine particle concentration.

In some embodiments, the at least one processor is configured to control the filtration system based on increasing a filtration strength in response to that the estimated ultra-fine particle concentration indicates that an ultra-fine particle concentration in air surrounding the vehicle system has increased and/or decreasing filtration strength in response to that the estimated ultra-fine particle concentration indicates that the ultra-fine particle concentration in air surrounding the vehicle system has decreased.

According to one embodiment, the cabin air system controller comprises at least one processor configured to obtain a measurement of a NOx concentration at a location of the vehicle, request an estimate of an ultra-fine particle concentration based on the NOx concentration (e.g. from a server computer (e.g. a cloud), i.e. a remote service, but also an onboard processor: correlation functions (lookup data) may also be pre-loaded and optionally kept updated to the vehicle, e.g., according to a pre-defined route, so that processing may, for example, be at the edge and not at the cloud. This has the benefit that real-time GPS location data need not be shared outside the vehicle for that purpose, increasing privacy. On the other hand, real-time information gives more fine-tuned concentration data and better control of the cabin air system.), receive an estimated ultra-fine particle concentration in response to the request, and control a cabin air system using the estimated ultra-fine particle concentration.

According to another aspect of the disclosure there is provided a vehicle comprising a cabin air system, the cabin air system comprising the aforementioned cabin air system controller.

In some embodiments, the vehicle further comprises a nitrogen oxide concentration sensor to obtain the measurement of a nitrogen oxide concentration at the location of the vehicle.

In some embodiments, the at least one processor is configured to receive nitrogen oxide concentration from a remote data source.

In some embodiments, the remote data source is at least one of a measuring point or from an air pollution map.

In some embodiments, the measurement of a nitrogen oxide concentration at the location of the vehicle is compared with or appended to the nitrogen oxide concentration received from the remote data source.

According to another aspect of the disclosure, there is provided a computer program element comprising program instructions, which, when executed by one or more processors, cause the one or more processors to perform the aforementioned method(s).

According to another aspect of the disclosure, there is provided computer-readable medium comprising program instructions, which, when executed by one or more processors, cause the one or more processors to perform the aforementioned method(s).

According to one embodiment, controlling the cabin air system comprises increasing a filter strength. The increasing of the filter strength may mean increasing an air airflow through a filter element. The increased airflow may be achieved via increasing a blower speed of a suction device, and/or opening one or more airflow control flaps in response to that the estimated ultra-fine particle concentration indicates that an ultra-fine particle concentration in air surrounding the vehicle system (e.g. air surrounding the vehicle or, in case that there is no vehicle sensor in the vehicle, a NOx sensor external to the vehicle) has increased and/or decreasing filter strength in response to that the estimated ultra-fine particle concentration indicates that the ultra-fine particle concentration in air surrounding the vehicle system has decreased. (So, for example, cabin air system air performance may be increased in case of higher pollution (in particular UFP concentrations) or, in case of lower pollution, its performance may be decreased to lower energy consumption in case of less pollution; it is also possible to keep a fresh air intake closed when driving through an area with high pollution).

It should be noted that embodiments described in context of the method system are analogously valid for the devices (vehicle, cabin air system controller, data processing system) and vice versa.

## BRIEF DESCRIPTION OF THE DRAWINGS

The disclosure will be better understood with reference to the detailed description when considered in conjunction with the non-limiting examples and the accompanying drawings, in which:

- FIG. 1 shows a vehicle with a cabin air system.

- FIG. 2 shows a flow diagram illustrating an estimation of UFP (ultra-fine particle) concentration based on nitrogen oxide.
- FIG. 3 and FIG. 4 show diagrams illustrating the correlation between NOx concentration and UFP concentration near a busy street.
- FIG. 5 shows a flow diagram illustrating a method for estimating an ultra-fine particle concentration.

## DETAILED DESCRIPTION

The following detailed description refers to the accompanying drawings that show, by way of illustration, specific details and embodiments in which the disclosure may be practiced. These embodiments are described in sufficient detail to enable those skilled in the art to practice the disclosure. Other embodiments may be utilized and structural, and logical changes may be made without departing from the scope of the disclosure. The various embodiments are not necessarily mutually exclusive, as some embodiments can be combined with one or more other embodiments to form new embodiments.

Embodiments described in the context of one of the devices or methods are analogously valid for the other devices or methods. Similarly, embodiments described in the context of a device are analogously valid for a vehicle or a method, and vice-versa.

Features that are described in the context of an embodiment may correspondingly be applicable to the same or similar features in the other embodiments. Features that are described in the context of an embodiment may correspondingly be applicable to the other embodiments, even if not explicitly described in these other embodiments. Furthermore, additions and/or combinations and/or alternatives as described for a feature in the context of an embodiment may correspondingly be applicable to the same or similar feature in the other embodiments.

In the context of various embodiments, the articles "a", "an" and "the" as used with regard to a feature or element include a reference to one or more of the features or elements.

As used herein, the term "and/or" includes any and all combinations of one or more of the associated listed items.

As used herein, the term "vehicle" includes a motor-powered vehicle, for example a vehicle having 2, 3, 4, or more wheels. Examples of the vehicle include a passenger car, a truck, a bus, a lorry, or a rail vehicle, for example, a locomotive. In some embodiments, the vehicle may be an electric vehicle or a hybrid (internal combustion engine and electrical) vehicle. In some embodiments, the vehicle may be an internal combustion engine (ICE) vehicle (i.e., non-hybrid). The term vehicle may further include autonomous vehicles

As used herein, the term "processor(s)", as used herein, includes one or more electrical circuits capable of processing data, i.e. processing circuits. A processor may include analog circuits or components, digital circuits or components, or hybrid circuits or components Any other kind of implementation of the respective functions which will be described in more detail below may also be understood as a "circuit" in accordance with an alternative embodiment. A digital circuit may be understood as any kind of a logic implementing entity, which may be special purpose circuitry or a processor executing software stored in a memory, or a firmware.

As used herein, the term "module" refers to, or forms part of, or include an Application Specific Integrated Circuit (ASIC); an electronic circuit; a combinational logic circuit; a field programmable gate array (FPGA); a processor (shared, dedicated, or group) that executes code; other suitable hardware components that provide the described functionality; or a combination of some or all of the above, such as in a system-on-chip. The term module may include memory (shared, dedicated, or group) that stores code executed by the processor.

As used herein, the term "data" may be understood to include information in any suitable analog or digital form, for example, provided as a file, a portion of a file, a set of files, a signal or stream, a portion of a signal or stream, a set of signals or streams, and the like. The term data, however, is not limited to the aforementioned examples and may take some forms and represent any information as understood in the art.

As used herein, the term "obtain", refers to the processor which actively obtains the inputs, or passively receives inputs from one or more sensors or data source. The term obtain may also refer to a processor, which receives or obtains inputs from a communication interface, e.g. a user interface. The processor may also receive or obtain the inputs via a memory, a register, and/or an analog-to-digital port.

As used herein, the term "ultrafine particle(s)" (UFPs) refers to particulate matter of nanoscale size, typically less than 0.1 micro-meters ($\mu$m) or 100 nano-meters (nm) in diameter. The term "ultrafine particle concentration" may be understood as "any data indicative of concentration"

As used herein, the term "sensor" may include at least one hardware sensor, at least one software sensor, or a combination of the at least one hardware and software sensors

In the following, embodiments will be described in detail.

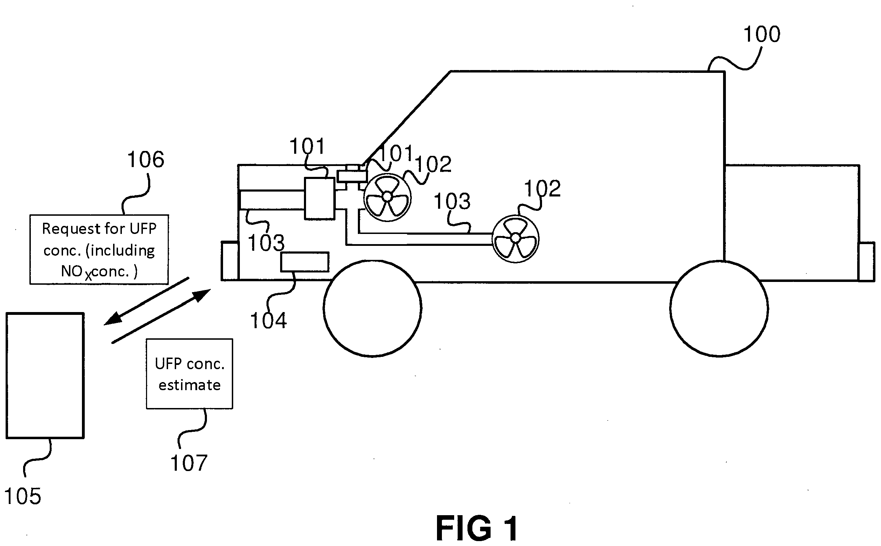

FIG. 1 shows a vehicle 100 with a cabin air system.

The cabin air system comprises one or more filters 101, fans 102, and ducts 103. In some embodiments, the filters 101, fans 102, and ducts 103 may be arranged in series to work together to provide and circulate air throughout the vehicle's cabin. The cabin air system is designed to remove pollutants, dust, and other harmful particles from the air while also regulating temperature and humidity levels. In particular, the cabin air system operates as a cabin air filtration system (and may be regarded as such). Similarly, the cabin air system may operate and be regarded as an in-vehicle ambient air filtration system (ambient air in the sense of air external to the vehicle). In some embodiments, the fans 102 may be configured to produce airflow containing the ultra-fine particles through the filters 101, i.e. the fans 102 may operate as suction devices/blowers to increase or decrease filtration strength of the filtration system. In some embodiments, the increase and decrease in filtration strength correspond respectively to an increased and decreased airflow drawn through the filters 101 over a period of time.

The cabin air system comprises a controller 104 which controls its operation by various control actions, e.g. sets fan speeds and the degree to which air vents and air ducts are opened. The controller 104 may for example measure interior air quality and perform corresponding control actions.

According to various embodiments, a more optimized cabin air (filtration) system is provided in that it takes into consideration not only the cabin air quality, but also the external air quality, in particular ultra-fine particle concentration to reduce health risks from ultra-fine particles (UFPs).

Air Quality data is typically available from public measurement stations (in particular measurements like PM10 and PM2.5 and also $NO/NO_2$ measurements etc.). So, a controller of a cabin air system could use this information. However, ultra-fine particle concentration may not be available by these measurement stations since, for example, a certain (local) measurement station only provides information about the current (local) pollution status regarding to defined parameters (such as PM10 or PM2.5 and gas concentrations) and thus, for example, no information about particles smaller than PM2.5 particles (which do not have the same sources as UFP and therefore do not sufficiently correlate with them to give accurate information about UFP concentration).

Moreover, it should be noted that due to their small size and mass, gravimetric or optical measurement methods, which are common for larger sizes of fine dust, are unsuitable for measuring UFP. The most important standardized methods for detecting and measuring the number concentration of UFP in air are particle counting methods and size classification based on electrical mobility. The measurement of a particle number size distribution allows an accurate assignment of particle sizes and the computational determination of a UFP number concentration over arbitrary particle size intervals. Mobility particle size spectrometers have a measurement uncertainty of up to about 10 percent in the range of the particle number concentration and down to a few percent in the range of the determined particle diameter. Below 10 nanometers (nm), greater uncertainties occur with the current state of the art. Continuous operation of mobility particle size spectrometers in the context of air monitoring requires regular maintenance and calibration by trained personnel. In the meantime, low-cost mobile measuring instruments that estimate the particle number concentration on the basis of an ion current measurement are also increasingly being found, but this is associated with reduced accuracy compared to condensation particle counters. Beyond the aforementioned real-time measuring instruments, it is possible in principle, albeit with greater technical effort, possible to examine UFP chemically or to produce electron microscope images.

So, it is difficult to measure UFP concentration, especially in a vehicle. Moreover, fewer measuring stations are typically available for UFP compared to PM2.5 and PM10.

Even in case the vehicle has a vehicle sensor for UFP concentration, it may be desirable to obtain information about UFP concentration from an additional source since otherwise, no (cross) check whether the sensor is working correctly is possible. So, an approach to estimate UFP concentration is desirable to ensure that the cabin air system (or one of its components such as a HEPA filter) is activated at the moment when UFP concentration is really high.

Therefore, according to various embodiments, an estimation method, which may include an estimation algorithm, may be used to determine the local UFP concentration at a location where the vehicle 100 is located. The determination of the local UFP concentration may be achieved by one or more onboard controllers of the vehicle 100, for example, controller 104, or may be via a server 105. In some embodiments, the server 105 may be a remote server may be a cloud server. In some embodiments, the determination includes an estimation of the local UFP concentration.

According to some embodiments, the estimation method uses a correlation between the nitrogen oxide concentration (e.g. NOx, where x may be 1 and/or 2) and at least one of the following data: a geographical location, a time parameter, such as a time of day, a day of the week and a season. In some embodiments, the geographical location may include a country, a suburb, a city, an airport, a road, a roadside, a mountain region. In some embodiments, the time parameter may include a time stamp, i.e. a computer time corresponding to that the data is obtained.

In an embodiment, the vehicle 100 may send a request 106 for the determination of an UPF concentration to the server 105. The vehicle 100 may measure or determine, e.g. by querying or consulting a reference database, the NOx concentration at its location and include it into the request 106. Alternatively, the vehicle may only include its location into a request 106 and the server 105 determines the NOx concentration itself. In any case, the server 105 or server arrangement may determine the UPF concentration by estimating the UPF concentration from the NOx concentration and the correlation between these two concentrations and sends the UPF concentration estimate 107 to the vehicle. In some embodiments, the server 105 may include a requestor processor to process the request 106, and a server processor to return the UPF concentration estimate 107.

In some embodiments, the location data may be obtained based on global positioning system (hereinafter referred to as GPS). By using GPS location data and nitrogen and NOx data, i.e. NO and/or NO2 concentration, e.g. from a vehicle sensor or from a remote data source, for example an official source or an air pollution map provider, the estimation method algorithm can calculate the UFP concentration. Thus, a variable that is currently difficult to measure, the UFP concentration, can be determined by using a "virtual sensor". It is appreciable that the data obtained from vehicle sensor may be compared with the data obtained from the remote data source. In some embodiments, the data obtained from the vehicle sensor may be augmented with the remote data, such that the accuracy or amount of information of both the local and/or remote data may be enhanced.

Further, from information about influence on UFP concentration of weather and/or ozone as well as the consideration of influencing factors like daytime, season and (as mentioned above) location (e.g. environment) may be used in the UFP concentration estimation to make a more accurate estimation of the UFP concentration.

Effects like solar radiation have an impact on new particle formation (NPF) that may be relevant for the determination of UFP concentration. In the formation of new particles, primary UFPs are mainly generated by emissions such as traffic emissions, industry emissions, emissions arising from burning processes. Secondary UFPs are mainly generated by NPF. For a more precise prediction, a distinction between primary and secondary UFPs may be made.

The NOx concentration based on which UFP concentration is estimated may be obtained by using a sensor. In some embodiments, the sensor may be a NOx sensor for sensing NO and $NO_2$. In some embodiments, such NOx sensor may be installed in the vehicle 100, i.e. as onboard sensors, or may be obtained from existing data from freely accessible measuring points or from manufacturers of air pollution maps to determine the NOx concentration. For the latter approach, no additional installation space in the vehicle 100 would have to be used for a corresponding sensor. In terms of sustainability, this may also save an additional component in the vehicle 100 and thus conserve resources.

In case an UFP sensor is provided in the vehicle 100, the UFP sensor may in some embodiments be a small, inexpensive and robust sensor for measuring UFP concentration.

In some embodiments, the estimation method may be used to monitor the correct functioning of the sensor and a malfunction of the sensor. The determination of malfunction of the sensor may indicate that a necessary replacement of the sensor is necessary. Such determination of malfunction may be reported to the end user (e.g. driver of the vehicle). This may provide a resource-saving option for lifetime monitoring of a sensor in terms of sustainability.

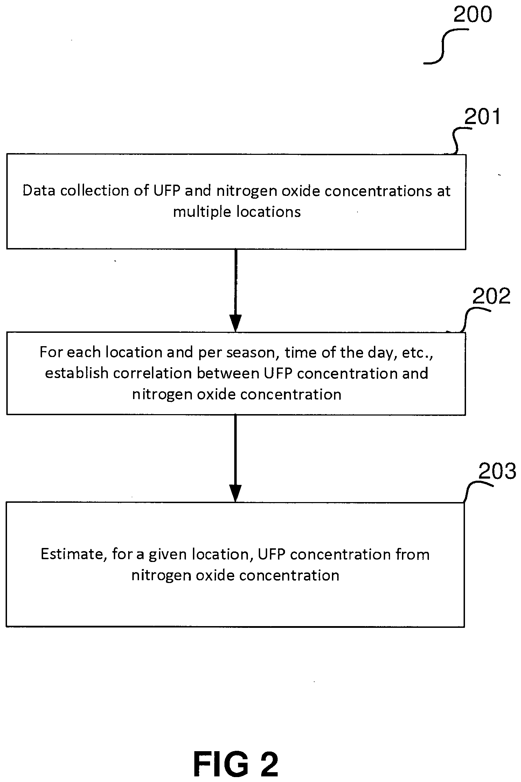

FIG. 2 shows a flow diagram 200 illustrating an estimation of UFP concentration based on NOx concentration, such as x = 1, i.e. NO concentration, and/or x= 2, $NO_2$ concentration.

In 201, at one or data measurements of UFP concentration and NOx concentration are collected over a relatively long period of time (e.g. months or even years) from a plurality of different geographical locations. It is appreciable that multiple data measurements may be collected at each of the plurality of different geographical locations.

In 202, a data processing unit may be arranged to obtain data measurements at each of the locations. The data processing unit may be a remote server or an onsite server. The data processing unit may be configured to perform one or more of the following functions:

- calculate statistical values such as average, mode, median, values of the data measurements over a predetermined period or time horizon. An example may be hourly averaged values for each day.
- determine or identify correlations of NOx concentration and UFP concentration based on one or more of the following criteria:

  ○ season- such as spring, summer, fall, winter
  ○ day of the week (or "weekday" and "weekend")
  ○ time of day (e.g. "night", "morning", "afternoon", "evening")
  ○ environment ("roadside", "mountain area", "urban area", "industrial area", "residential area")

In some embodiments, the correlation may be expressed mathematically based on a linear dependency as follows:

$$UFP\_concentration = a * nitrogen\_oxide\_concentration + b$$

where a and b are correlation constants. The data processing unit may be configured to determine the real numbers a and b based on each of the above criteria or for each combination thereof.

In 203, the data processing unit or another data processing unit, possibly in the vehicle 100 such as the controller 104, may use the determined correlations to determine UFP concentration based on nitrogen concentration NOx at a location. In some embodiments, the location, may be different from the other locations at which the measurements where obtained. In some embodiments, the UFP concentration may be determined by using the determined values of a and b for the season, day of the week, time of the day and/or environment for which the UFP concentration should be determined (and the nitrogen concentration was measured).

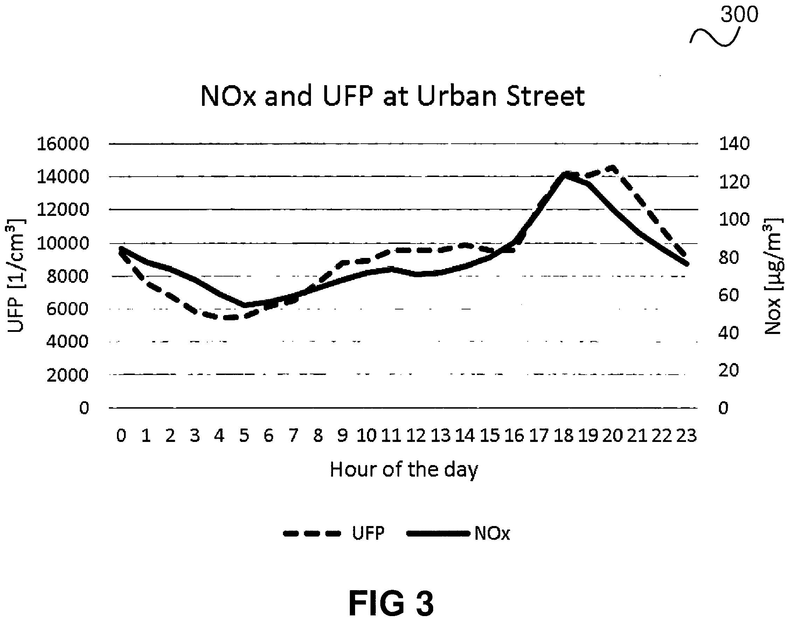

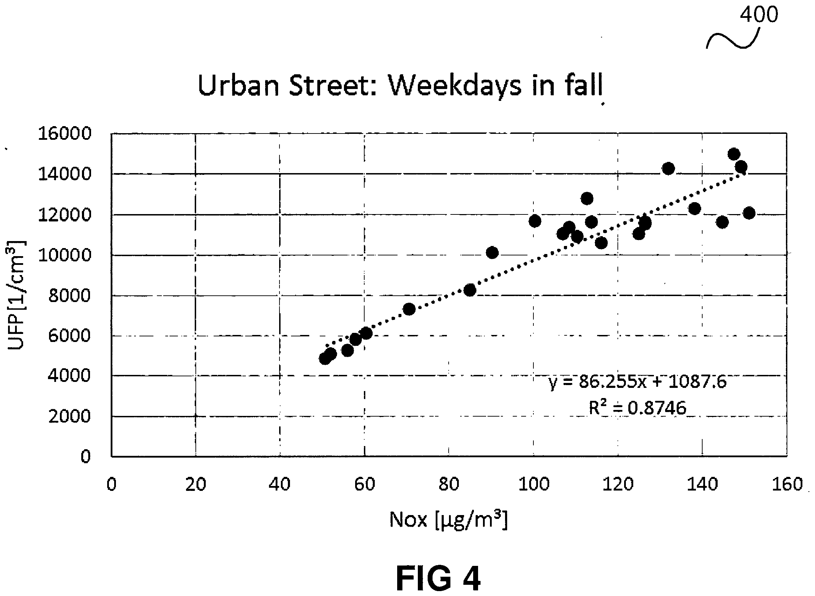

FIG. 3 and FIG. 4 show diagrams 300, 400 illustrating the correlation between NOx concentration and UFP concentration near an urban street, which may be relatively busy.

The diagram 300 of FIG. 3 includes hourly averages for the NOx and the UFP concentration of a weekday in fall season.

The diagram 400 of FIG. 4 shows that UFP concentration may be well described by a linear function from the NOx concentration. The constant a is determined to be 86.255 and the constant b is determined to be 1087.6. FIG. 4 illustrates the linear function obtained through a curve fitting model, i.e. a regression model, with the $R^2$ value of 0.8746.

In summary, according to various embodiments, a method is provided as illustrated in 5.

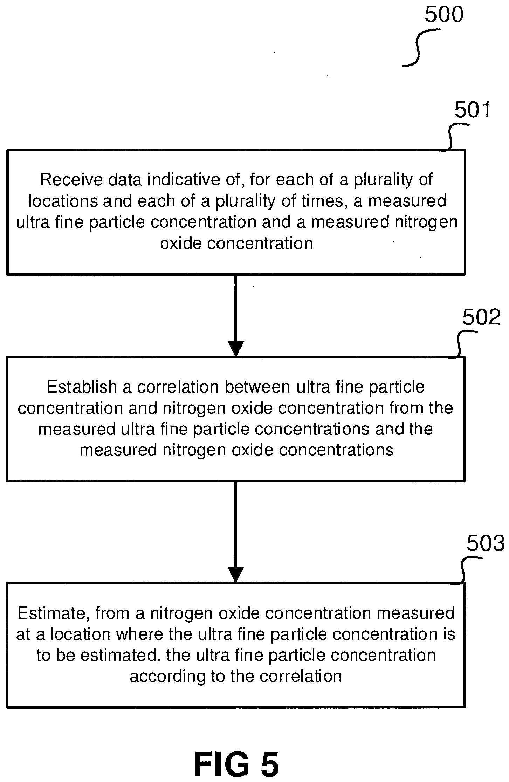

According to one embodiment, a method is provided as illustrated in FIG. 5.

FIG. 5 shows a flow diagram illustrating a method for estimating an ultra-fine particle concentration.

In 501, data is received indicative of, for each of a plurality of locations and each of a plurality of times, a measured ultra-fine particle concentration and a measured NOx concentration (this may include measuring the ultra-fine particle concentrations and the measured NOx concentrations and may, for example, be performed by a server computer). The measured ultra-fine particle concentration and the measured nitrogen oxide concentration may be correlated to/associated with at least one location of a plurality of locations, and a corresponding time of a plurality of times. As an example, the measured ultra-fine particle concentration and the measured nitrogen oxide concentration may be received from a road, in the afternoon 3pm (off-peak traffic hours) or in the morning 8am (peak traffic hours).

In 502 a correlation between ultra-fine particle concentration and NOx concentration is established from the measured ultra-fine particle concentrations and the measured NOx concentrations.

In 503, from a NOx concentration measured at a location where the ultra-fine particle concentration is to be estimated, the ultra-fine particle concentration is estimated according to the correlation (this may include interpolation between correlations (or corresponding formulas or models, like of the parameters a and b in case of the linear model described above) in case the UFP concentration should be determined at a location for which no correlation was established).

According to various embodiments, in other words, UFP concentration is estimated by exploiting correlation of UFP concentration with NOx concentration. Thus, the estimation approach of FIG. 5 allows a precise estimation of the UFP concentration at a certain location by means of simple measurement technology, such as existing NOx sensors, without the need for complex measurement technology.

Besides being used, for example, for controlling a cabin air system, the estimated UPF concentration may also be displayed to a user via, for example, a user interface. So, instead of displaying air quality (AQ) on a dashboard or mobile application only based on measured values (either coming from external AQ measurement stations or from in-cabin sensor measurements the UPF estimation approach of FIG. 5 may be used to calculate an in-cabin AQ and display it without using additional sensors.

The estimated UFP concentration or a derivate thereof, such as an air quality index, which may be calculated on board or in a server, such as a remote cloud server, may also be made available at an API (Application Programming Interface) so that it can be accessed by other services. In some embodiments, the other services may include onboard or smartphone apps.

The methods described herein may be performed and the various processing or computation units and the devices and computing entities described herein may be implemented by one or more circuits. In an embodiment, a "circuit" may be understood as any kind of a logic implementing entity, which may be hardware, software, firmware, or any combination thereof. Thus, in an embodiment, a "circuit" may be a hard-wired logic circuit or a programmable logic circuit such as a programmable processor, e.g. a microprocessor. A "circuit" may also be software being implemented or executed by a processor, e.g. any kind of computer program, e.g. a computer program using a virtual machine code. Any other kind of implementation of the respective functions which are described herein may also be understood as a "circuit" in accordance with an alternative embodiment. A device (e.g. server) or arrangement (e.g. cabin system controller and server) which performs the method according to any of the embodiments may for example include one or more communication interfaces (to receive and send the various data and information e.g. position information, NOx concentration, UFP concentration estimate and control signals for the cabin air system) processing units and memories.

While the disclosure has been particularly shown and described with reference to specific embodiments, it should be understood by those skilled in the art that various changes in form and detail may be made therein without departing from the spirit and scope of the disclosure as defined by the appended claims. The scope of the disclosure is thus indicated by the appended claims and all changes which come within the meaning and range of equivalency of the claims are therefore intended to be embraced.

**Claims**

1. A method (500) for estimating an ultra-fine particle concentration, comprising:

   receiving (501) data indicative of, a measured ultra-fine particle concentration and a measured nitrogen oxide concentration, the measured ultra-fine particle concentration and the measured nitrogen oxide concentration correlated to each location of a plurality of locations and corresponding time of a plurality of times;
   establishing (502) a correlation between ultra-fine particle concentration and nitrogen oxide concentration from the measured ultra-fine particle concentrations and the measured nitrogen oxide concentrations; and
   estimating (503), from a nitrogen oxide concentration measured at a location where the ultra-fine particle concentration is to be estimated, the ultra-fine particle concentration according to the correlation.

2. The method (500) of claim 1, wherein the nitrogen concentration is a nitrogen monoxide concentration, a nitrogen dioxide concentration, or a concentration of nitrogen monoxide and nitrogen dioxide.

3. The method (500) of claim 1 or claim 2, comprising receiving, by a server (105), a request (106) for an estimate of the ultra-fine particle concentration (107), wherein the request (106) indicates the nitrogen oxide concentration, and providing the estimated ultra-fine particle concentration (107) in response to the request (106).

4. The method (500) of any one of claims 1 to 3, comprising measuring, by a vehicle sensor, the nitrogen oxide concentration at the location where the ultra-fine particle concentration is to be estimated and/or comprising requesting the nitrogen oxide concentration at the location where the ultra-fine particle concentration is to be estimated from a database.

5. The method (500) of any one of claims 1 to 4, comprising controlling a vehicle system using the estimated ultra-fine particle concentration (107),
   preferably wherein the vehicle system is a cabin air system.

6. The method (500) of claim 5, wherein controlling the cabin air system comprises increasing a filtration strength in response to that the estimated ultra-fine particle concentration (107) indicates that an ultra-fine particle concentration in air surrounding the vehicle system has increased and/or decreasing filtration strength in response to that the estimated ultra-fine particle concentration (107) indicates that the ultra-fine particle concentration in air surrounding the vehicle system has decreased.

7. The method (500) of any one of claims 1 to 5, wherein estimating the ultra-fine particle concentration further comprises depending on the location where the ultra-fine particle concentration is to be estimated, a time parameter, and information about a weather at the location where the ultra-fine particle concentration is to be estimated.

8. A data processing system configured to perform the method of any one of claims 1 to 7.

9. A cabin air system controller (104) for a cabin air system of a vehicle (100), the cabin air system controller (104) comprising at least one processor configured to

   obtain a measurement of a nitrogen oxide concentration at a location of the vehicle;
   sending a request (106), from the controller (104), to a server (105), for an estimate of an ultra-fine particle concentration based on the nitrogen oxide concentration;
   receive, by the controller (104), an estimated ultra-fine particle concentration (107) in response to the request (106), the estimated ultra-fine particle concentration (107) determined according to any one of the method of claims 1 to 7; and
   control a filtration system of the cabin air system based on the estimated ultra-fine particle concentration.

10. The cabin air system controller (104) of claim 9, wherein the at least one processor is configured to control the filtration system based on increasing a filtration strength in response to that the estimated ultra-fine particle concentration (107) indicates that an ultra-fine particle concentration in air surrounding the vehicle system has increased and/or decreasing filtration strength in response to that the estimated ultra-fine particle concentration (107) indicates that the ultra-fine particle concentration in air surrounding the vehicle system has decreased.

11. A vehicle (100) comprising a cabin air system, the cabin air system comprising a cabin air system controller (104) of claim 9 or claim 10.

12. The vehicle (100) of claim 11, further comprising a nitrogen oxide concentration sensor to obtain the measurement of a nitrogen oxide concentration at the location of the vehicle (100).

13. The vehicle (100) of claim 11 or 12, wherein the at least one processor is configured to receive nitrogen oxide concentration from a remote data source,

    preferably wherein the remote data source is at least one of a measuring point or from an air pollution map, and/or wherein the measurement of a nitrogen oxide concentration at the location of the vehicle (100) is compared with or appended to the nitrogen oxide concentration received from the remote data source.

14. A computer program element comprising program instructions, which, when executed by one or more processors, cause the one or more processors to perform the method of any one of claims 1 to 7.

15. A computer-readable medium comprising program instructions, which, when executed by one or more processors, cause the one or more processors to perform the method of any one of claims 1 to 7.

**FIG 1**

200

201

Data collection of UFP and nitrogen oxide concentrations at multiple locations

202

For each location and per season, time of the day, etc., establish correlation between UFP concentration and nitrogen oxide concentration

203

Estimate, for a given location, UFP concentration from nitrogen oxide concentration

# FIG 2

300

## NOx and UFP at Urban Street

UFP [1/cm³] vs NOx [μg/m³] — Hour of the day

- - - UFP    ——— NOx

**FIG 3**

EP 4 506 194 A1

400

Urban Street: Weekdays in fall

$y = 86.255x + 1087.6$
$R^2 = 0.8746$

UFP [1/cm³]

Nox [µg/m³]

**FIG 4**

500

501

Receive data indicative of, for each of a plurality of locations and each of a plurality of times, a measured ultra fine particle concentration and a measured nitrogen oxide concentration

502

Establish a correlation between ultra fine particle concentration and nitrogen oxide concentration from the measured ultra fine particle concentrations and the measured nitrogen oxide concentrations

503

Estimate, from a nitrogen oxide concentration measured at a location where the ultra fine particle concentration is to be estimated, the ultra fine particle concentration according to the correlation

**FIG 5**

Europäisches Patentamt

European Patent Office

Office européen des brevets

# EUROPEAN SEARCH REPORT

**Application Number**

EP 23 31 5307

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LONGLEY ET AL: "Using NO"x and CO monitoring data to indicate fine aerosol number concentrations and emission factors in three UK conurbations", ATMOSPHERIC ENVIRONMENT, ELSEVIER, AMSTERDAM, NL, vol. 39, no. 28, 1 September 2005 (2005-09-01), pages 5157-5169, XP005028333, ISSN: 1352-2310, DOI: 10.1016/J.ATMOSENV.2005.05.017 | 1-4,7,8, 14,15 | INV. B60H3/06 G01N15/06 |
| Y | * page 5161, left-hand column, line 4 – page 5161, right-hand column, line 12 * * page 6162, right-hand column, line 6 – page 6162, right-hand column, line 12 * * page 5165, right-hand column, line 41 – page 5165, right-hand column, line 44 * | 5,6,9-13 | |
| X | US 2019/317003 A1 (TAKASU RYOZO [JP]) 17 October 2019 (2019-10-17) | 1-4,7 | |
| Y | * paragraph [0005] – paragraph [0045] * | 5,6,9-13 | TECHNICAL FIELDS SEARCHED (IPC) |
| Y | CN 111 873 758 A (CHENGDU WANYOU FILTER CO LTD) 3 November 2020 (2020-11-03) | 5,6,9-13 | B60H |
| A | * paragraph [0034] – paragraph [0039]; figure 1 * | 1-4,7,8, 14,15 | G01N |

The present search report has been drawn up for all claims

| Place of search | Date of completion of the search | Examiner |
|---|---|---|
| The Hague | 6 February 2024 | Stavroulakis, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

[X] All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION**
**SHEET B**

The Search Division considers that the present European patent application does not comply with the requirements of unity of invention and relates to several inventions or groups of inventions, namely:

    **1. claims: 1-4, 7, 8, 14, 15**

        **Measuring nitrogen oxide by a vehicle sensor, thereby improving the mobility of the measurement system.**
        **---**

    **2. claims: 5, 6, 9-13**

        **Adjusting filtering in a cabin air system based on an estimated ultra-fine particle concentration, thereby improving air quality.**
        **---**

## ANNEX TO THE EUROPEAN SEARCH REPORT
## ON EUROPEAN PATENT APPLICATION NO.

**EP 23 31 5307**

This annex lists the patent family members relating to the patent documents cited in the above-mentioned European search report.
The members are as contained in the European Patent Office EDP file on
The European Patent Office is in no way liable for these particulars which are merely given for the purpose of information.

**06-02-2024**

| Patent document cited in search report | Publication date | Patent family member(s) | | Publication date |
|---|---|---|---|---|
| US 2019317003 A1 | 17-10-2019 | CN | 110168347 A | 23-08-2019 |
| | | JP | 6880756 B2 | 02-06-2021 |
| | | JP | 2018112534 A | 19-07-2018 |
| | | US | 2019317003 A1 | 17-10-2019 |
| | | WO | 2018131470 A1 | 19-07-2018 |
| CN 111873758 A | 03-11-2020 | NONE | | |

EPO FORM P0459

For more details about this annex : see Official Journal of the European Patent Office, No. 12/82